# EUROPEAN PATENT APPLICATION

(11) **EP 0 360 566 A2**
(43) Date of publication of application: **28.03.1990**
(21) Application number: 89309531.5
(22) Date of filing: 19.09.1989
(51) Int. Cl.: C07D 263/58, A61K 31/42, C07D 413/04, A61K 31/44

(54) **Lipoxygenase/cyclooxygenase inhibiting benzoxazolones**

(30) Priority: 19.09.1988 JP 234539/88
(71) Applicant: PFIZER INC., New York, N.Y. 10017 (US)
(72) Inventor: Nakane, Masami, Nagoya-shi Aichi-Ken, 466 (JP); Kokura, Toshihide, Handa-shi Aichi-Ken, 475 (JP); Ito, Fumitaka, Chita-gun Aichi-Ken, 470-23 (JP)
(74) Representative: Wood, David John

(57) **Abstract**

A compound of the formula 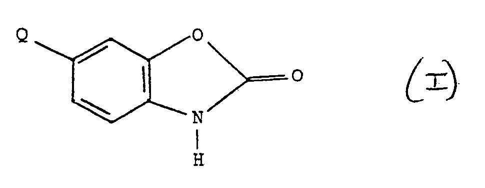 wherein Q is as defined below, and the pharmaceutically acceptable salts thereof. The compounds are inhibitors of lipoxygenase and cyclooxygenase, and are useful in the treatment of allergic and inflammatory conditions.

## Description

The present invention relates to certain benzoxazolone compounds having an aryl or heteroaryl substituted amino group at the 6-position and to certain 6-aryl or heteroaryl ether derivatives of benzoxazolone. These compounds are dual inhibitors of LO (lipoxygenase) and CO (cyclooxygenase) enzymes, and are useful as antiallergy and antiinflammatory agents in mammalian subjects.

Co-pending U.S. Patent Application Ser. No. 129,020 by Kitaura et al., refers to benzoxazolone compounds having an alkylamino group at the 6-position, and states that such compounds are LO and CO inhibitors.

PCT Patent Application WO 85/01289 refers to benzoxazolone and benzothiazolone derivatives containing halogen, lower alkyl or lower alkoxy substituents on the benzo ring and a hetero atom containing side chain, and states that they are useful for the treatment of inflammatory conditions and thrombosis.

This invention relates to novel benzoxazolone compounds of the formula: 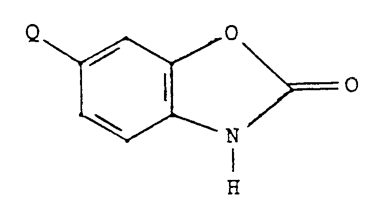 or a pharmaceutically acceptable acid addition salt thereof, wherein
X is sulfur or oxygen;
R₁ is hydrogen or C₁-C₄ alkyl;
R₂ is a heteroaryl selected from furyl, thienyl, pyridyl, pyrrolyl, imidazolyl or indazolyl; or group of the formula: 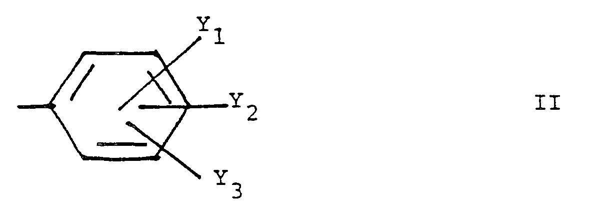 wherein
Y₁ is hydrogen, hydrozyl, halo, C₁-C₄ alkyl,
C₁-C₄ alkoxy, phenoxy, carboxymethyl or
C₁-C₃ dialkylamino; Y₂ is hydrogen or
C₁-C₄ alkyl; Y₃ is hydrogen or
C₁-C₄ alkyl; and
R₃ is phenyl or pyridyl.

Preferred are those compounds having the formula I wherein R₁ is hydrogen and R₂ is

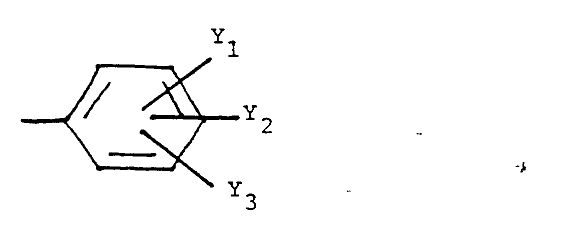

Within this preferred group, particularly preferred compounds are those wherein Y₂ and Y₃ are each hydrogen; and those wherein Y₁ and Y₂ are each C₁-C₄ alkyl and Y₃ is hydrogen. More preferably, when Y₁ or Y₂ or Y₃ is C₁-C₄ alkyl, said C₁-C₄ alkyl is methyl; and when Y₁ is halo, said halo is chloro.

Specific compounds of the invention are:
[6-(5-Indolyl)amino] -2-benzoxazolinone;
[6-(5-Benzothienyl)amino]-2-benzoxazolinone;
[6-(2,6-Dichloro-4-dimethylaminophenyl)amino]-2 benzoxazolinone;
[6-(5-Imidazolyl) amino]-2-benzoxazolinone;
[6-(5-Benzofuryl)amino]-2-benzoxazolinone;
[6-(2-Hydroxypyrid-5-yl)amino]-2-benzoxazolinone;
[6-(3-Furyl)amino]-2-benzoxazolinone;
[6-(2,3-Dimethyl-4-methoxyphenyl)amino]-2-benzoxazolinone;
[6-(4-Oxo-6-chromanonyl)amino]2-benzoxazolinone; and
[6-(2-Dimethylaminophenyl)amino]-2-benzoxazolinone.

When R₂ is heteroaryl, it is preferably pyridyl. A preferred compound is one wherein R₁ is hydrogen and R₂ is 2-pyridyl.

Another preferred group of the present compounds are those having the formula wherein Q is -XR₃.

The present invention also relates to the pharmaceutically acceptable acid addition salts of the compounds of the formula 1. Examples of such salts are those formed from acids which form non-toxic acid addition salts, e.g., the hydrochloride, hydrobromide, sulfate or bisulfate, phosphate or acid phosphate, acetate, citrate, fumarate, gluconate, lactate, maleate, succinate, tartrate, methanesulfonate, benzene sulfonate, toluenesulfonate, and formate salts.

The present invention further relates to a pharmaceutical composition for treating an allergic or inflammatory condition in a mammal, especially man, comprising a pharmaceutically acceptable carrier or diluent and a compound of formula 1 or a pharmaceutically acceptable salt thereof.

The present invention further relates to a method for treating an allergic or inflammatory condition in a mammal, especially man, comprising administering to a mammal in need of such treatment an antiallergy or antiinflammatory effective amount of a compound of formula I, or a pharmaceutically acceptable salt thereof.

The present invention further relates to a method of inhibiting the action of lipoxygenase or cyclooxygenase in a mammal, especially man, which comprises administering to a mammal a lipoxygenase or cyclooxygenase inhibiting amount of a compound of formula I, or a pharmaceutically acceptable salt thereof.

The novel compounds of formula I may be prepared by the reaction sequences described below.

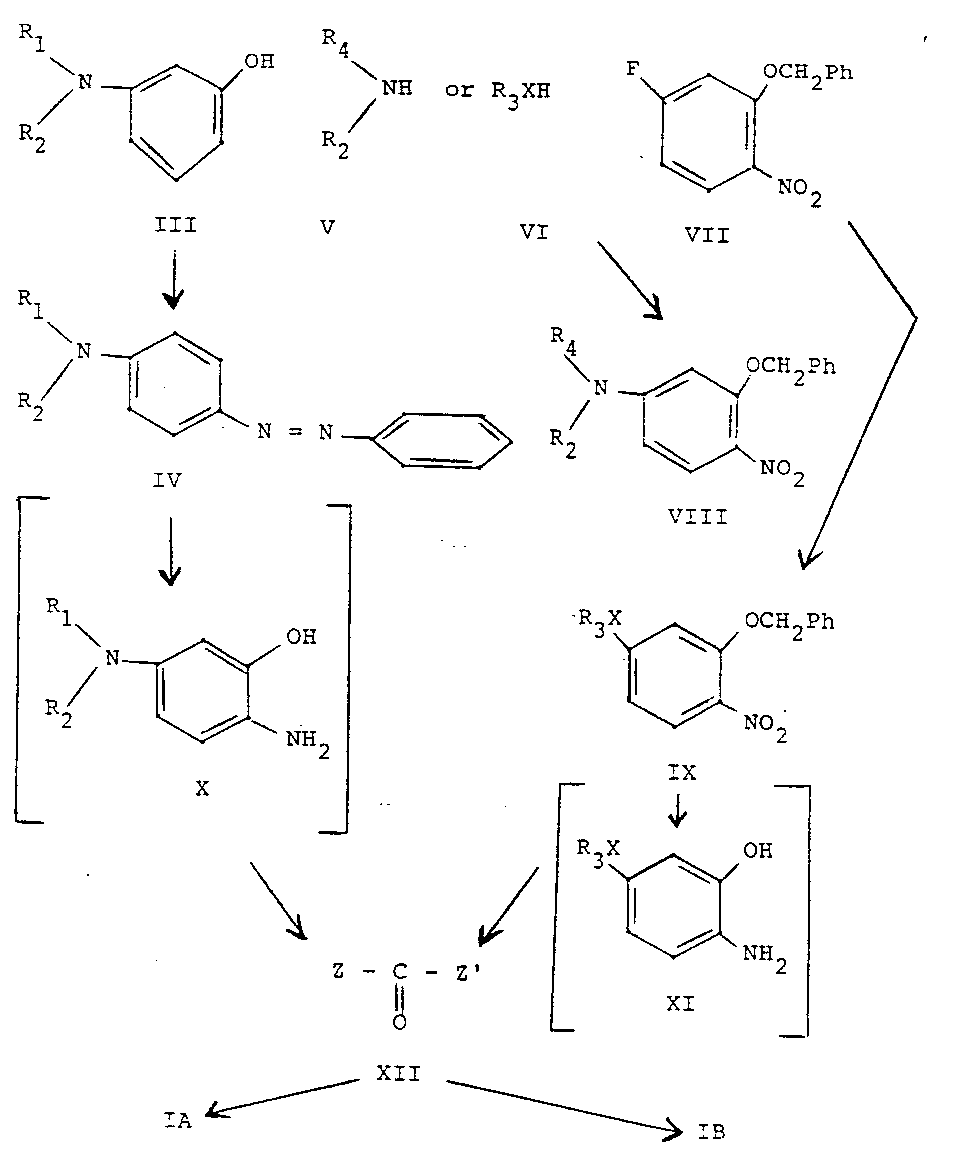

In the above scheme, the label "IA" is used to designate compounds of formula I wherein Q is -NR₁R₂ and R₁ and R₂ are as defined above, and the label "IB" is used to designate compounds of formula I wherein Q is -XR₃ and R₃ is as defined above.

In one preferred sequence, a hydroxyaniline derivative of formula III, wherein R₁ and R₂ are as defined above, is coupled with a benzenediazonium salt to provide a compound of the formula IV. The required benzenediazonium salt such as e.g., benzenediazonium chloride can be obtained according to standard procedures. The coupling reaction is carried out in a reaction-inert solvent, e.g., water. The reagents are usually combined at a temperature between 0°C and 5°C and then allowed to warm to ambient temperature (typically over 1 hour at 25°C). The starting hydroxyanilines of formula II are either known compounds or readily obtainable using standard synthetic methods.

Compounds of the formula IA wherein R₁ is hydrogen are conveniently prepared by a different sequence. This synthesis requires an amine of the formula V wherein R₂ is as previously defined and R₄ is a suitable amino protecting group such as formyl or t-butoxycarbonyl. The amine of formula V is reacted with 2-benzyloxy-4-fluoronitrobenzene (VII) to prepare a compound of the formula VIII. Similarly, compound of the formula VI, wherein X and R₃ are as previously defined, can be reacted with compounds of the formula VII to give a compound of the formula IX.

The reaction of the amine of formula V with 2-benzyloxy-4-fluoronitrobenzene is carried out in a reaction-inert solvent, e.g. N,N-dimethylformamide, in the presence of a base such as potassium t-butoxide or potassium carbonate. The reaction temperature is dependent upon the nature of the amino protecting group. For example, when R₄ is formyl, higher temperatures from about 100° to 120°C can be employed in combination with potassium carbonate. When R₄ is t-butoxycarbonyl, the reaction can be carried out at ambient temperature in the presence of potassium t-butoxide. When R₄ is formyl or any other base labile group, deprotection occurs in the product (VIII) with resultant elimination of the R₄ group at this stage. When R₄ is t-butoxycarbonyl, its removal is preferably conducted in an acidic medium once the final product IA is obtained, in which case R₁ is still t-BOC.

In preparing compounds of the formula IX, substantially the same reaction conditions are employed as for the synthesis of compound of the formula VIII. Preferably, this reaction is carried out at a temperature from about 0 to 25°C in N,N-dimethylformamide in the presence of potassium t-butoxide, for a period of about 0.5 to 2.0 hours.

Hydrogenation of any of compounds IV, VIII and IX followed by reaction of the intermediate X or XI with a compound of the formula XII wherein Z and Z′ are both suitable leaving groups affords a product of the formula IA or IB. Typically, hydrogenation is accomplished by using palladium carbon in a polar solvent, e.g. ethyl acetate, at low hydrogen pressures (1 to 3 atm) and ambient temperature. Hydrogenation is usually complete within a few hours. The intermediate bifunctional compounds of the formulae X and XI can be isolated and purified conventionally, e.g. recrystallization or chromatography. It is, however, more convenient not to isolate this product but to contact it with the reagent of formula XII in situ. This one-step process is conveniently carried out following the hydrogenation reaction by adding an approximately equimolar amount of a compound of formula XII with or without a solvent. Representative examples of compounds of formula XII are phosgene, dimethyl carbonate, diethyl carbonate, urea, N,N-carbodiimidazole, methyl chloroformate and trichloromethyl chloroformate. Trichloromethyl chloroformate is preferred.

The solvent for this coupling should be either the same as was used for hydrogenation or one that is both miscible with the hydrogenation solvent and inert. The preferred medium is ethyl acetate. The reaction is conducted at a temperature between 0° and 25°C, preferably at about 5°C, at which temperature the reaction is substantially complete in about 5 to 60 minutes. The desired benzoxazolones IA and IB are then isolated and purified by conventional methods, such as recrystallization or chromatography.

Compounds of the formula IA wherein R₁ is methyl are preferably prepared by methylating the 5 amino position of the corresponding compounds of the formula IA wherein R₁ is hydrogen. This is conveniently done by forming an immonium derivative of the compound of formula IA with formaldehyde and reducing the immonium intermediate. A reagent suited for this purpose is sodium borohydride, used in excess (e.g. 5 moles/mole of substrate). The compound of formula IA, formaldehyde and sodium borohydride are combined at a temperature of about 0 to 25°C in inert solvent such as tetrahydrofuran. The product can be isolated and purified conventionally.

The pharmaceutically acceptable salts of the novel compounds of the present invention are readily prepared by contacting said compounds with a stoichiometric amount of an appropriate mineral or organic acid in either aqueous solution or in a suitable organic solvent. The salts may then be obtained by precipitation or by evaporation of the solvent.

The compounds of the present invention are inhibitors of cyclooxygenase and lipoxygenase enzymes. Their activity has been demonstrated by an assay using rat peritoneal cavity resident cells which determines the effect of said compounds on the metabolism of arachidonic acid.

In this test, some preferred compounds indicate low IC50 values, in the range of 1 to 10 M, with respect to inhibition of both lipoxygenase and cyclooxygenase.

The ability of the compounds of the present invention to inhibit both enzymes make them useful for controlling the symptoms induced by the endogenous metabolites arising from arachidonic acid in a mammalian subject. The compounds are therefore valuable in the prevention and treatment of disease states in which the accumulation of an arachidonic acid metabolite is the causative factor, e.g., allergic bronchial asthma, skin disorders, rheumatoid arthritis, osteoarthritis, and thrombosis.

The antiinflammatory activity of the compounds of the present invention can be determined according to the standard carrageenin-induced rat foot edema test (C. A. Winter et al., Proc. Soc. Exp. Biol. 111, p. 544, 1962), where the same preferred compounds are found to show over 50% inhibitions at the 50 mg/kg dose level when administered orally.

Thus, compounds of the formula I and their pharmaceutically acceptable salts are of particular use in the treatment or alleviation of allergic or inflammatory conditions in a human subject.

For treatment of the various conditions described above, the compounds of formula I and their pharmaceutically acceptable salts can be administered to a human subject either alone, or, preferably, in combination with a pharmaceutically acceptable carrier or diluent, according to standard pharmaceutical practice. The compounds can be administered by a variety of conventional routes of administration including orally, parentally and by inhalation. When the compounds are administered orally, the dose range will be from about 0.1 to 20 mg/kg body weight of the subject to be treated per day, preferably from about 0.1 to 1.0 mg/kg per day, in single or divided doses. If parental administration is desired, then an effective dose will be from about 0.1 to 1.0 mg/kg body weight of the subject to be treated per day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of formula I can be administered, for example, in the form of tablets, powders, lozenges, syrups or capsules, or as an aqueous solution or suspension. In the case of tablets for oral use, carriers which are commonly used include lactose and corn starch, and lubricating agent such as magnesium stearate are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added. For intramuscular, intraperitoneal, subcutaneous and intravenous use, sterile solutions of the active ingredient are usually prepared, and the pH of the solutions suitably adjusted and buffered. For intravenous use, the total concentration of solutes should be controlled to make the preparation isotonic.

The present invention is illustrated by the following examples. However, it should be understood that the invention is not limited to the specific details of these examples. Proton nuclear magnetic resonance spectra (NMR) were measured at 270 MHz unless otherwise indicated for solutions in perdeuterodimethyl sulfoxide (DMSO-d₆) and peak positions are expressed in parts per million (ppm) downfield from tetramethysilane. The peak shapes are denoted as follows: s, singlet; n, multiplet; b, broad.

### Example 1

### 6-Phenylamino-2-benzoxazolone

2-Phenyldiazo-5-phenylaminophenol (1.0 g, 3.46 mmol) was hydrogenated over 5% palladium/carbon (200 mg) in ethyl acetate for 1.5 hours. To this hydrogenated solution was added trichloromethyl chloroformate (3.46 m moles) at 5°C. Stirring was continued at room temperature for 10 minutes. Water (5 ml) was added to the reaction mixture and it was stirred at room temperature for 30 minutes. The palladium carbon was filtered off, and saturated aqueous sodium bicarbonate solution was added to the filtrate. The organic material was extracted with ethyl acetate (30 ml x 3). The combined ethyl acetate extracts were washed with brine, dried over MgSO₄, and concentrated at reduced pressure to yield crude product as an oil. The oil was chromatographed on silica gel, eluting with ethyl acetate/hexane (1:1 by volume) to give 496 mg of the title compound: m.p. 167°-168°C.
IR (Nujiol): 3500, 3450, 3280, 1740, 1650 cm⁻¹
¹H NMR: 11.38 (br, s, 1H), 8.07 (s, 1H), 7.20 (t, 2H), 6.75-7.01 (m, 6H).

| | | | |
|---|---|---|---|
| Analysis: Calcd. for C₁₃H₁₀N₂O₂: | C, 69.01; | H, 4.46; | N, 12.38%. |
| Found: | C, 68.94; | H, 4.45; | N, 12.35%. |

### Example 2-6

Following the procedure of Example 1, and starting with the appropriate reagents, the following compounds were prepared:

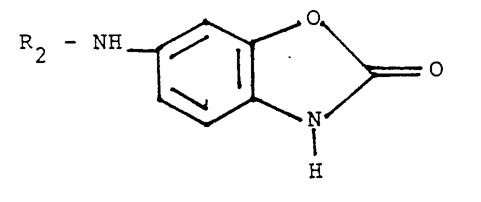

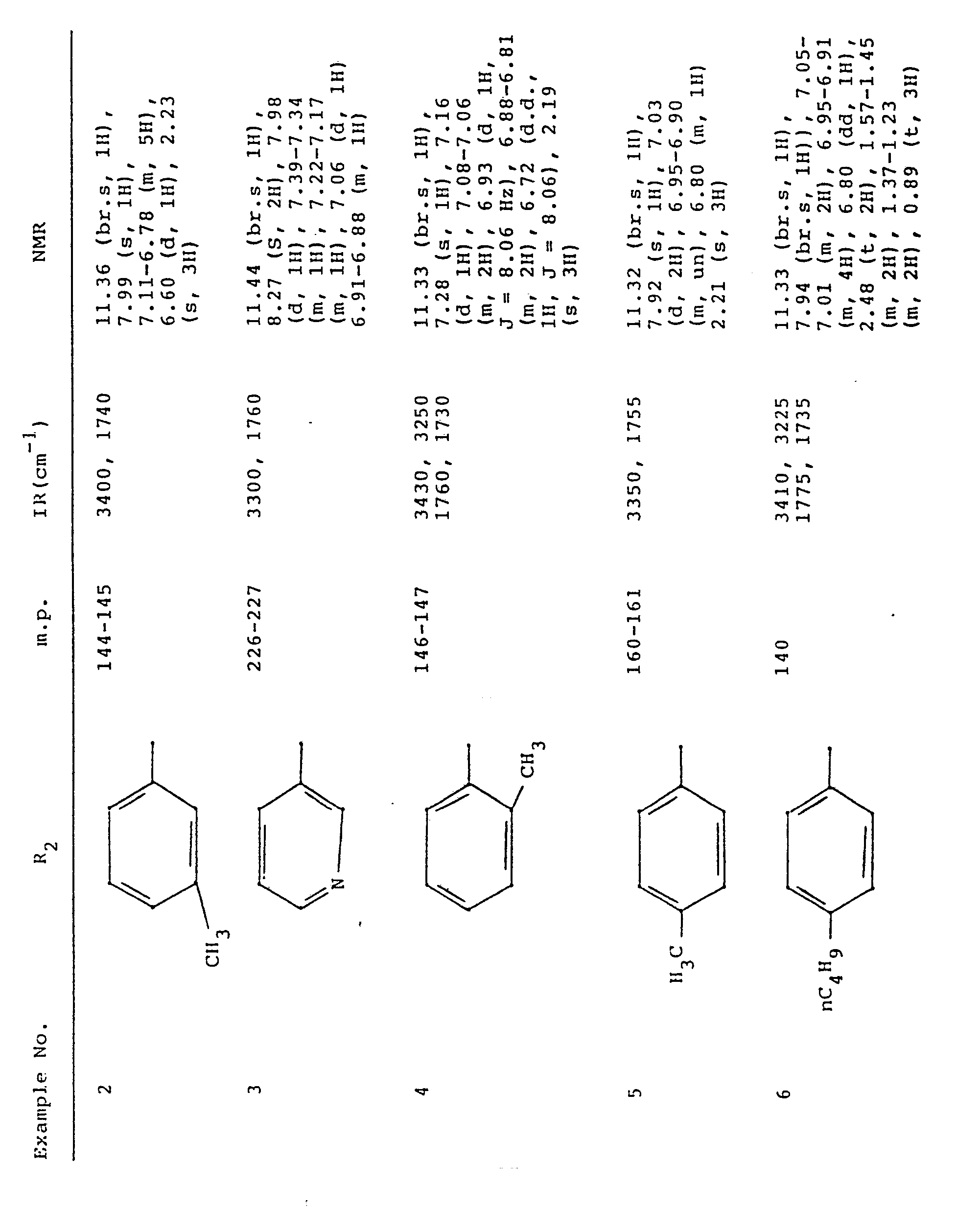

### Example 7

### 6-(4-chlorophenyl) amino-1-benzoxazolone

6-(4-chlorophenyl) amino-2-benzyloxynitrobenzene (2.07 g, 5.83 mmoles) was hydrogenated over 5% palladium/carbon (1.00 g) in ethyl acetate for 2 hours. To this hydrogenated solution was added trichloromethyl chloroformate (5.83 mmoles) at 5°C. Stirring was continued at room temperature for 30 minutes. Water (20 ml) was added to the reaction mixture and it was stirred at room temperature for 15 minutes. The palladium/carbon was filtered off, and saturated aqueous sodium bicarbonate solution was added to the filtrate. The organic material was extracted with ethyl acetate (50 ml x 2). The combined ethyl acetate extracts were washed with brine, dried over MgSO₄, and concentrated at a reduced pressure to yield crude product as a solid.

The crude product was recrystallized from ethyl acetate-n-hexane to give 560 mg of the title compound:
mp 189°-191°C.
IR (Nujiol): 3440, 1760 cm⁻¹,
¹H NMR: 11.41 (br.s, 1H), 8.22 (s, 1H), 7.23-7.19 (m, 2H), 7.02-6.95 (m, 4H), 6.86 (dd, 1H)

| | | | |
|---|---|---|---|
| Analysis: Calcd. for C₁₃H₁₉N₂O₂Cl: | C, 59, 89; | H, 3.48; | N, 10.75% |
| Found: | C, 59.96; | H, 3.47; | N, 10.80% |

### Examples 8-17

Following the procedure of Example 7, and starting with the appropriate reagents, the following compounds were prepared:

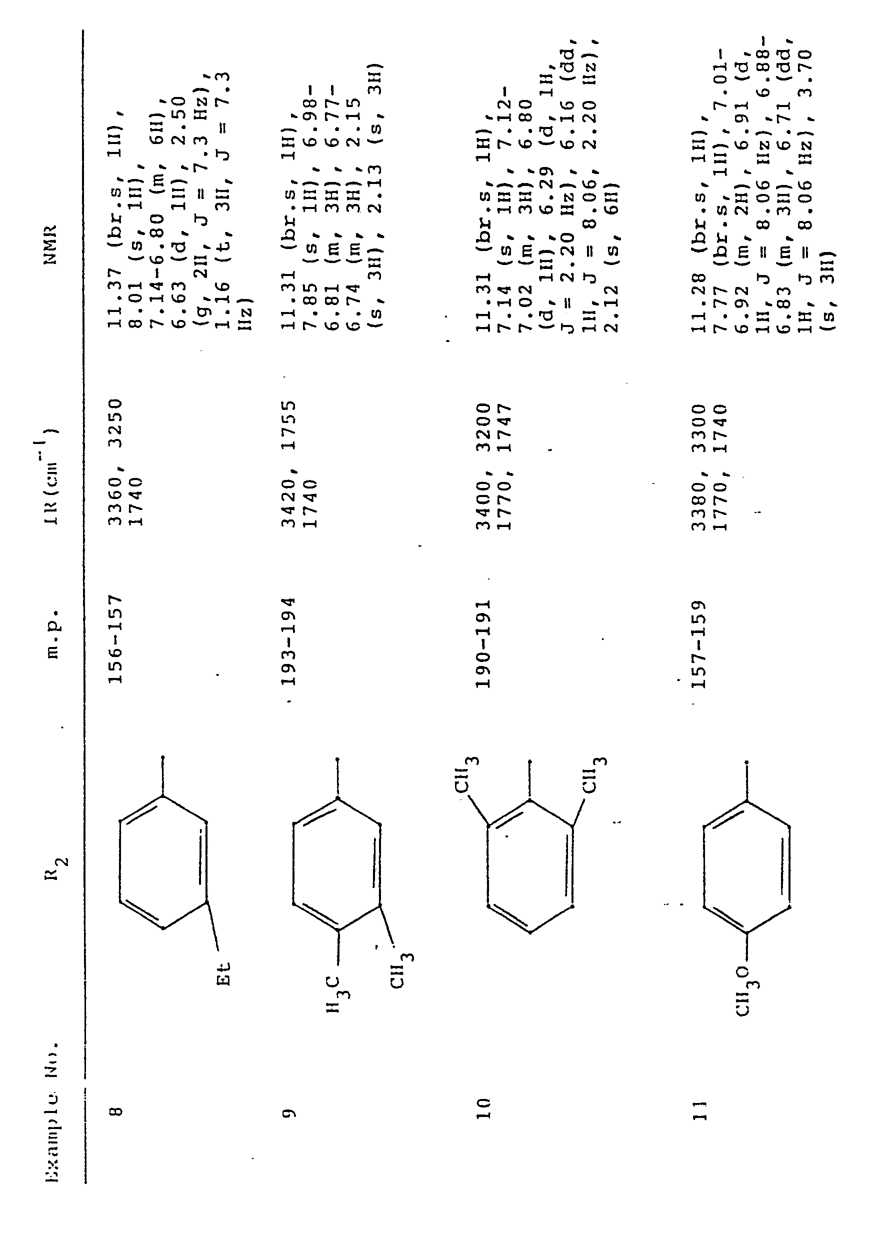

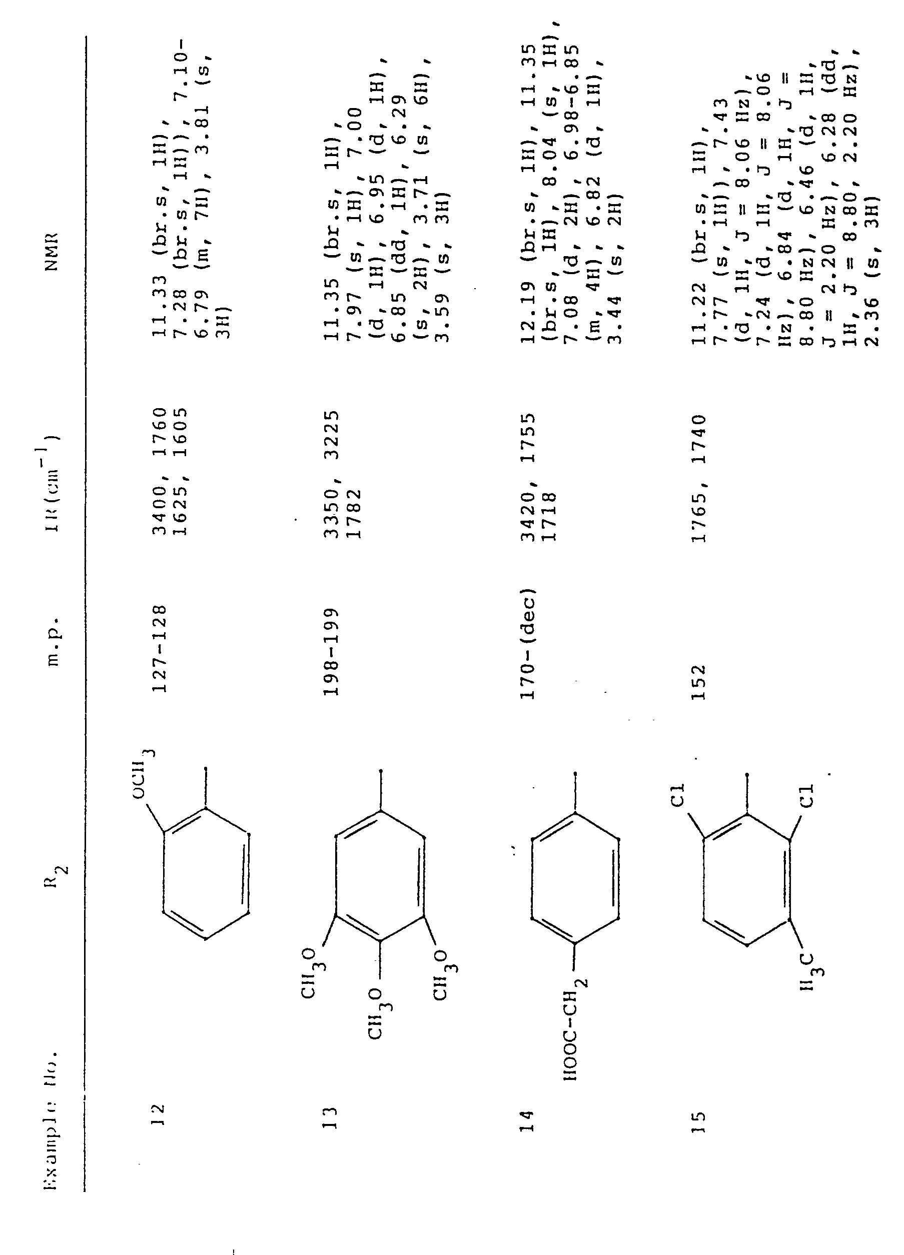

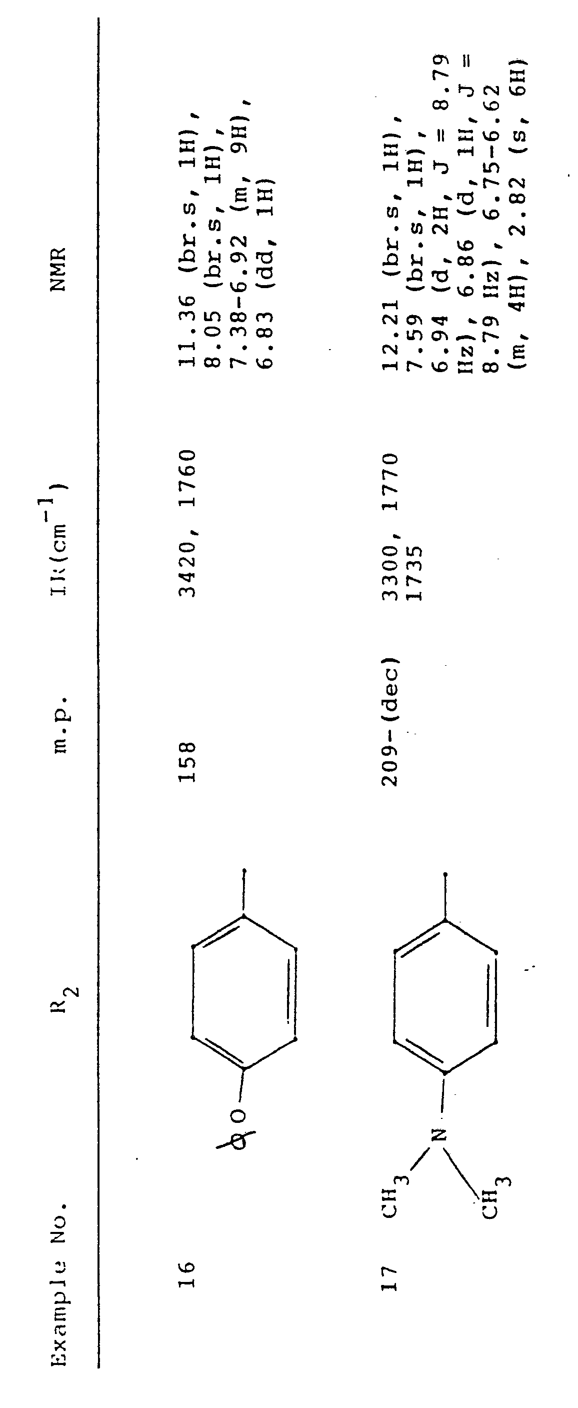

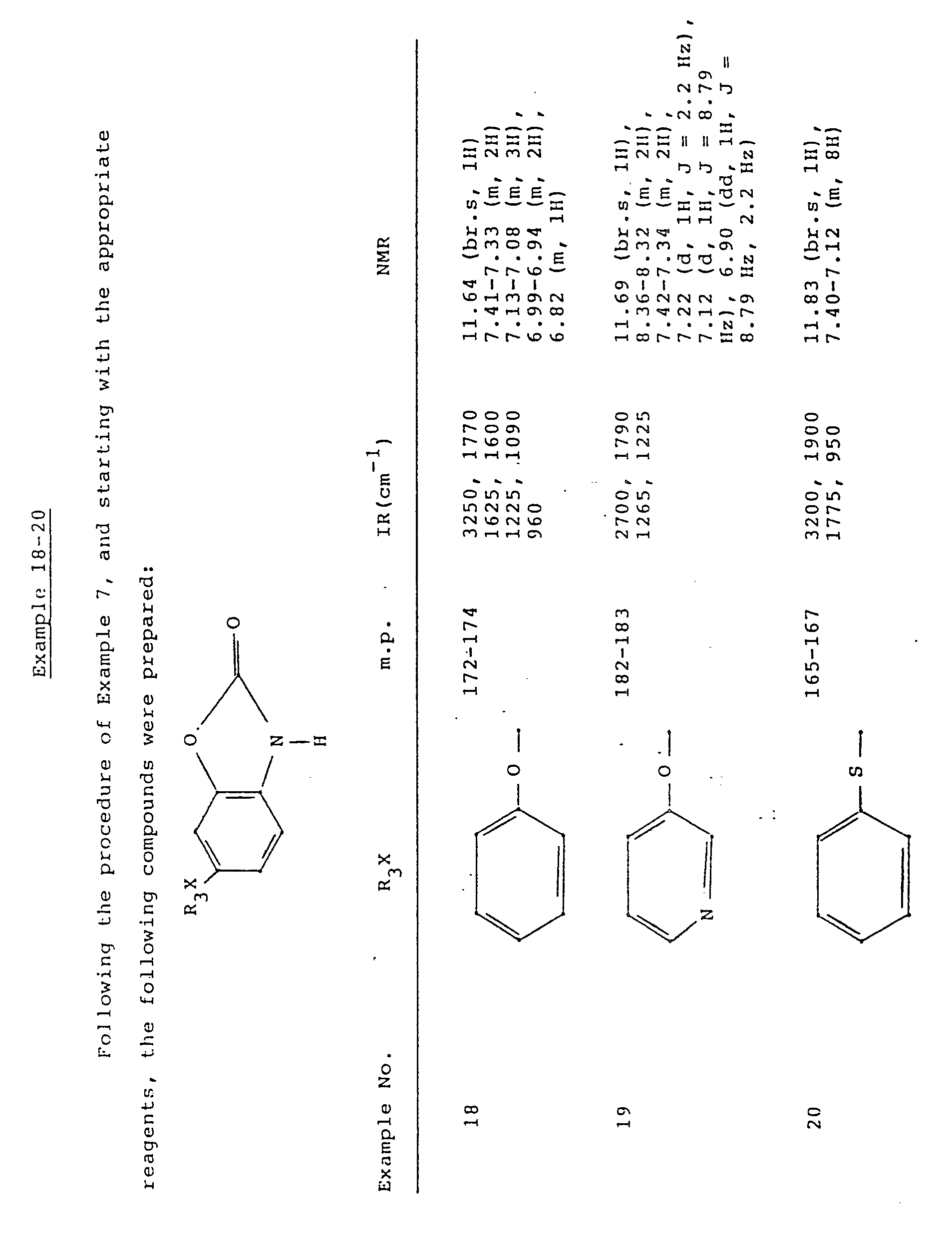

### Example 21

### 6-(2,6-Diethylphenyl)amino-2-benzoxazolone

N-t-Butoxycarbonyl-6-(2,6-diethylphenyl)amino-2-benzyloxy nitrobenzene (1.9 g, 4.09 mmoles) was hydrogenated over 5% palladium/carbon (0.5 g) in ethyl acetate (50 ml) for 2 hours. The palladium/carbon was removed by filtration, the filtrate was cooled to 5°C, and triethylamine (4.10 mmoles) was added to the filtrate. Trichloromethyl chloroformate (4.09 mmoles) was added at 5°C. Stirring was continued at room temperature for 30 minutes. Water (20 ml) was added to the reaction mixture, and it was stirred at room temperature for 15 minutes. Saturated aqueous sodium bicarbonate solution was added and the product was extracted with ethyl acetate (120 ml x 2). The combined ethyl acetate extracts were washed with brine and dried over MgSO₄. Removal of the solvent gave a crude oil which was purified by chromatography on silica gel, eluting with ethyl acetate-n-hexane, to yield N-t-butoxycarbonyl-6-(2,6-diethylphenyl)amino-2-benzoxazolone (1.21 g, 80%). The NMR spectrum showed peaks at 11.58 (br.s, 1H), 7.32-7.19 (m, 4H), 6.96 (d, 1H, J = 8.8 Hz), 6.70 (dd, 1H, J = 8.8 Hz), 2.44 (m, 4H), 1.36 (2, 9H) , 1.09 (t, 6H) ppm.

To a stirred solution of N-t-butoxycarbonyl-6-(2,6-diethylphenyl)amino-2-benzoxazolone (4.1 g, 11.1 mmoles) in 50 ml of dichloromethane at room temperature was added trifluoroacetic acid (111 mmoles). Stirring was continued for 2 hours. When the solvent was removed, water was added. The organic material was extracted with ethyl acetate (50 ml x 2). The combined extracts were washed with brine and dried over MgSO₄. The dried solution was evaporated, and the residue was recrystallized from ethanol to give N-trifluoroacetyl- 6-(2,6-diethylphenyl)amino-2-benzoxazolone (2.51 g, 59%).

To a solution of this product (1.36 g, 3.6 mmoles) in tetrahydrofuran (14 ml) was added a solution of lithium hydroxide monohydrate (749 mg, 17.9 mmoles) in water (3.5 ml). Stirring was continued at 60°C for 2 hours. The solvent was removed, and water was added to the resulting oil. The organic material was extracted with ethyl acetate (20 ml x 2). The combined extracts were washed with brine and dried over MgSO₄. The dried solution was concentrated, and then chromatographed over silica gel using 1:1 ethyl acetate/hexane as eluent to afford 0.92 g (90%) of the title compound.
m.p. 140-141°C
IR (KBr): 3400, 3200, 3000, 1760, 1645 cm⁻¹
¹H NMR: 11.1 (br.s, 1H), 7.2 (br.s, 1H), 7.15 (s, 3H) , 6.78 (d, 1H, J = 8.1 Hz) , 6.26 (d, 1H, J = 2.2 Hz), 6.13 (dd, 1H, J = 8.1, 2.2 Hz), 2.47 (g, 4H, J =7.3 Hz), 1.04 (t, 6H, J = 7.3 Hz)

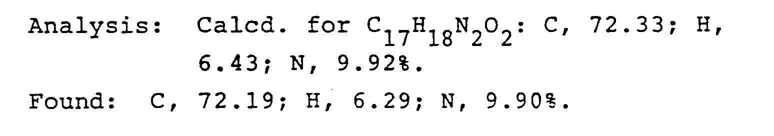

### Example 22

### 6-(2-N, N-Dimethylphenyl)amino-2-benzoxazolone

According to the methods of Example 21, by starting with appropriate N-t-butoxycarbonylaniline derivatives, the title compound was prepared.

### Example 23

### 6-(N-Methyl-N′-phenyl)amino-2-benzoxazolone

To a mixed solution of 3M sulfuric acid and 40% aqueous formaldehyde was added dropwise a suspension of sodium borohydride and 6-(phenylamino)-2-benzoxazolone at -10°C. Dichloroethane was then added and the aqueous phase was made basic by the addition of 5% sodium bicarbonate solution. The organic layer was separated and washed with brine. The dried solution was evaporated at a reduced pressure, and the residue obtained was recrystallized from dichloromethane-ether to give of the title compound.
m.p. 136-137°C
IR (KBr): 3290, 1790, 1740, 1600 cm⁻¹.
¹H NMR: 11.52 (br.s, 1H), 7.24-7.18 (m, 2H), 7.08-7.02 (m, 2H), 6.88-6.79 (m, 4H), 3.22 (s, 3H)

### Preparation A

### 2-Phenyldiazo-5-phenylaminophenol

Aniline (29.6 mmoles) was dissolved in aqueous hydrochloric acid solution (a mixture of 5.7 ml concentrated hydrochloric acid and 6 ml water) and cooled to 0°C. To this solution was added an aqueous sodium nitrite solution (2.06 g, NaNO₂ in 29 ml water) at 0°-5°C. Stirring was continued for 30 minutes.

m-Hydroxydiphenylamine (5.03 g, 27.5 mmoles) was dissolved in aqueous sodium hydroxide solution (3.4 g in 34 ml water). To this solution was added the above solution at 0° - 5°C over a 30 minute period. Stirring was continued at room temperature for 1 hour. The organic material was extracted with ethyl acetate (20 ml x 2). The combined extracts were washed with brine, dried over MgSO₄, and concentrated at a reduced pressure to yield crude product as an oil.

The oil was chromatographed on silica gel, eluting with chloroform to give 2.93 g of the title compound.
m.p. 176° - 177°C
¹H NMR: 13.26 (br.s, 1H) 9.11 (br.s, 1H) , 7.80-7.05 (m, 1H) 6.68 (dd, 1H, J = 8.8, 2.2 Hz), 6.48 (br.s, 1H)

In a similar manner, by starting with appropriate diphenylanilines and employing the procedures of Preparation A, the following 2-phenyldiazo-5-phenylaminophenols were prepared:
2-phenyldiazo-5-(3-tolyl) aminophenol,
2-phenyldiazo-5-(2-tolyl) aminophenol,
2-phenyldiazo-5-(4-tolyl) aminophenol,
2-phenyldiazo-5-(4-n-butyl) aminophenol, and
2-phenyldiazo-5-(2-pyridyl) aminophenol.

### Preparation B

### 6-(4-Chlorophenyl)amino-2-benzyloxynitrobenzene

Under a nitrogen atmosphere, a mixture of N-formyl-p-chloroaniline (3.47 g, 22.3 mmoles), 4-fluoro-2-benzyloxy-nitrobenzene (6.06 g, 24.5 mmoles) and potassium carbonate (6.16 g, 44.6 mmoles) in dimethylformamide (30 ml) was heated at 120°C for 2 hours. The reaction mixture was cooled, water (50 ml) was added, and the resulting precipitates were collected. Recrystallization from acetone gave the purified title compound, 3.41 g (43%).
m.p. 176° - 177°C
¹H NMR: 9.24 (s, 1H), 7.94 (d, 1H, J = 8.8 Hz), 7.50-7.33 (m, 7H), 7.17-7.13 (m, 2H), 6.76 (d, 1H, J = 2.2 Hz) , 6.62 (dd, 1H, J = 8.8, 2.2 Hz) , 5.27 (s, 2H)

In a similar manner, by starting with the appropriate N-formylaniline derivatives and employing the procedures of Preparation B, the following benzyloxynitrobenzenes were prepared:

4-(3-ethylphenyl)amino-2-benzyloxynitrobenzene,
4-(3,4-dimethylphenyl)amino-2-benzyloxynitrobenzene,
4-(2,6-dimethylphenyl)amino-2-benzyloxynitrobenzene,
4-(4-methoxyphenyl)amino-2-benzyloxynitrobenzene,
4-(2-methoxyphenyl)amino-2-benzyloxynitrobenzene,
4-(3,4,5-trimethoxyphenyl)amino-2-benzyloxynitrobenzene,
4-(4-carboxymethylphenyl)amino-2-benzyloxynitrobenzene,
4-(2,6-dichloro-3-methylphenyl)amino-2-benzyloxynitrobenzene,
4(4-phenoxyphenyl)amino-2-benzyloxynitrobenzene,
4-(4-N,N-dimethylaminophenyl)amino-2-benzyloxynitrobenzene.

### Preparation C

### N-t-Butoxycarbonyl-6,4-(2,6-diethylphenyl)amino-2-benzyloxynitrobenzene

Under a nitrogen atmosphere, a mixture of N-t-butoxycarbonyl-2,6-diethylaniline (1.89 g, 7.59 mmoles), 4-fluoro-2-benzyloxynitrobenzene (1.70 g, 7.59 mmoles) and potassium t-butoxide (0.85 g, 7.59 mmoles) in dimethylformamide (12 ml) was stirred at room temperature for 9 hours. The dimethylformamide was removed in vacuo from the reaction mixture and water was added. The organic material was extracted with ethyl acetate (30 ml x 2). The combined ethyl acetate extracts were washed with brine, dried over MgSO₄, and concentrated to yield a crude product, which was crystallized from acetone to give 3.40 g (95%) of the title compound.
IR (KBr): 3000, 1720 cm⁻¹
¹H NMR: 7.88 (d, 1H, J = 9.5 Hz), 7.41-7.19 (m, 9H), 6.73 (1H, dd, J = 9.5 Hz, 2.2 Hz), 5.15 (s, 2H), 2.3 (m, 4H), 1.35 (s, 9H), 1.03 (t, 6H), ppm.

In a similar manner, by starting with an appropriate N-t-butoxycarbonylaniline derivative and employing the procedures of Preparation C, N-t-butoxycarbonyl-4-(2,N,N-dimethylaminophenyl)amino-2-benzyloxynitrobenzene was obtained.

## Claims

1. A compound of the formula: 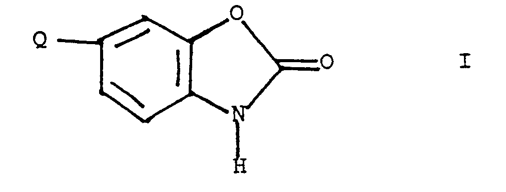 or a pharmaceutically acceptable salt thereof, wherein
Q is -NR₁R₂ or -XR₃;
X is sulfur or oxygen;
R₁ is hydrogen or (C₁-C₄) alkyl;
R₂ is 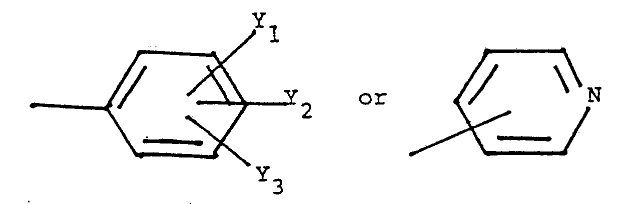 wherein Y₁ is hydrogen, hydroxyl, halo, (C₁-C₄) alkoxy, phenoxy, carboxymethyl or (C₁-C₃) dialkylamino; Y₂ is hydrogen, (C₁-C₄) alkyl or halo; Y₃ is hydrogen or (C₁-C₄) alkyl; and
R₃ is phenyl or pyridyl.

2. A compound according to claim 1, wherein Q is -NR₁R₂, R₁ is hydrogen and R₂ is

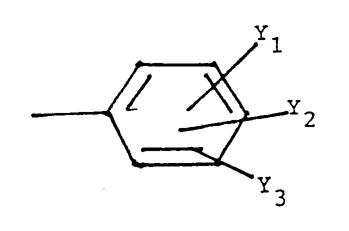

3. A compound according to claim 2, wherein Y₂ and Y₃ are each hydrogen.

4. A compound according to claim 2 wherein Y₁ and Y₂ are independently (C₁-C₄) alkyl and Y₃ is hydrogen;

5. A compound according to claim 1, wherein Q is -NR₁R₂, R₁ is hydrogen and R₂ is

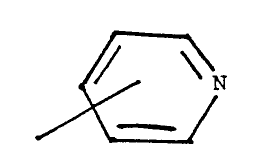

6. A compound according to claim 1, wherein Q is -NR₁R₂, R₁ is methyl and R₂ is

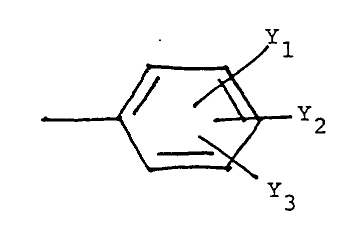

7. A compound according to claim 1, wherein Q is -XR₃ and X is sulfur.

8. A compound according to claim 1, wherein Q is -XR₃ and X is oxygen.

9. A pharmaceutical composition for the treatment of allergic or inflammatory conditions in a mammal, comprising an antiallergic or antiinflammatory effective amount of a compound according to claim 1, or a pharmaceutically acceptable salt thereof, and a pharmaceutically acceptable carrier.

## Claims (Claims for the following Contracting State(s): - ES, GR)

1. A process for making a compound of the formula 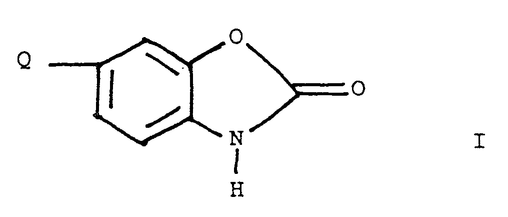 or a pharmaceutically acceptable acid addition salt thereof, wherein
Q is -NR₁R₂ or -XR₃;
X is sulfur or oxygen;
R₁ is hydrogen or C₁-C₄ alkyl;
R₂ is 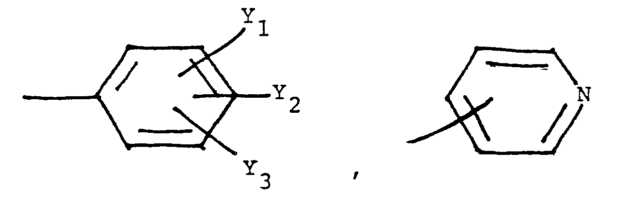 wherein Y₁ is hydrogen, hydroxyl, halo, C₁-C₄ alkoxy, phenoxy, carboxymethyl or C₁-C₃ dialkylamino; Y₂ is hydrogen, C₁-C₄ alkyl or halo; and Y₃ is hydrogen or C₁-C₄ alkyl, comprising: (a) reacting a compound of the formula 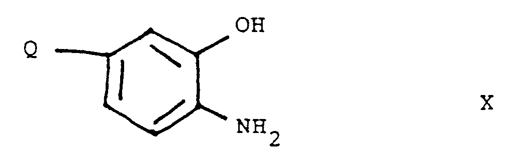 with a compound of the formula ZCOZ′, wherein Z and Z′ are the same or different and each of Z and Z′ is a suitable leaving group: and (b) if desired, making a pharmaceutically acceptable acid addition salt of said compound of formula I obtained in step (a).

2. A process according to claim 1 wherein Q is -NR₁R₂, R₁ is hydrogen, and R₂ is

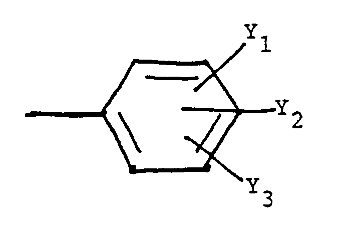

3. A process according to claim 2, wherein Y₂ and Y₃ are each hydrogen.

4. A process according to claim 2, wherein Y₁ and Y₂ are independently (C₁-C₄) alkyl and Y₃ is hydrogen.

5. A process according to claim 1, wherein Q is -NR₁R₂, R₁ is hydrogen and R₂ is

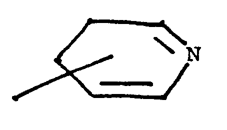

6. A process according to claim 1, wherein Q is -NR₁R₂, R₁ is methyl, and R₂ is

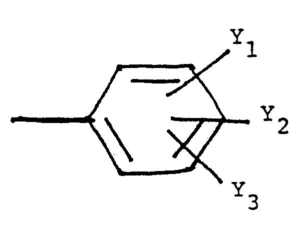

7. A process according to claim 1, wherein Q is -XR₃ and X is sulfur.

8. A process according to claim 1, wherein Q is -XR₃ and X is oxygen.

9. A process according to claim 1, wherein said compound of formula X is obtained by hydrogenating a compound of the formula 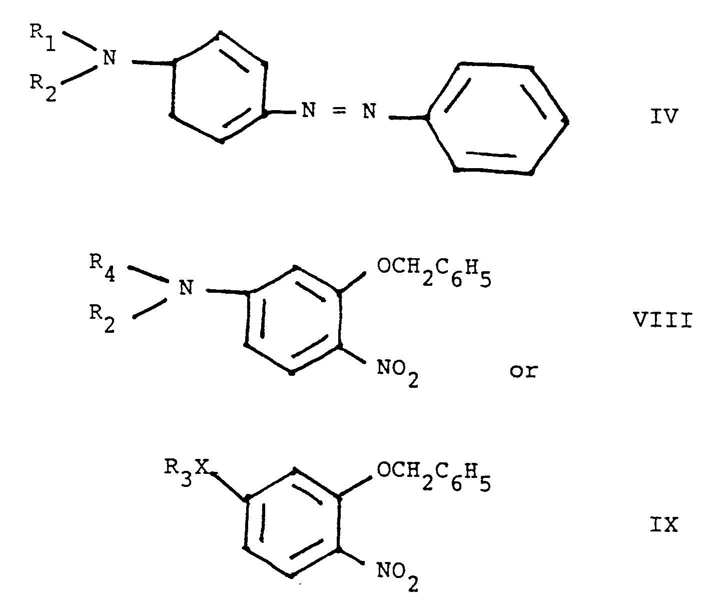 wherein R₄ is a suitable amino protecting group.

10. A process according to claim 9, wherein the amino protecting group is formyl or t-butoxycarbonyl.

11. A process according to any one of the preceding claims, wherein said compound of the formula ZCOZ′ is selected from the group consisting of phosgene, dimethyl carbonate, diethyl carbonate, urea, N,N-carbodiimidazole, methyl chloroformate and trichloromethyl chloroformate.

12. A process according to claim 9, wherein said compound of formula X is not isolated but is reacted in situ with said compound of the formula ZCOZ′.
